(19) Europäisches Patentamt
European Patent Office
Office européen des brevets

(11) EP 1 262 565 A2

(12) EUROPEAN PATENT APPLICATION

(43) Date of publication:
04.12.2002 Bulletin 2002/49

(51) Int Cl.7: C12Q 1/68

(21) Application number: 02253662.7

(22) Date of filing: 23.05.2002

(84) Designated Contracting States:
AT BE CH CY DE DK ES FI FR GB GR IE IT LI LU
MC NL PT SE TR
Designated Extension States:
AL LT LV MK RO SI

(30) Priority: 25.05.2001 US 293425 P

(71) Applicant: Pfizer Products Inc.
Groton, Connecticut 06340 (US)

(72) Inventors:
• Affourtit, Jason Patrick
Groton, Connecticut 06340 (US)
• Nelson, Darcy Lynn
Groton, Connecticut 06340 (US)
• Seymour, Albert Barnes
Groton, Connecticut 06340 (US)
• Webb, Suzin Marie
Groton, Connecticut 06340 (US)

(74) Representative: Hayles, James Richard et al
Pfizer Limited,
European Patents Department,
Ramsgate Road
Sandwich, Kent CT13 9NJ (GB)

(54) Genetic polymorphisms in the human neurokinin 1 receptor gene and their uses in diagnosis and treatment of diseases

(57) This invention provides nucleic acid segments derived from the neurokinin 1 receptor (TACR 1) locus of the human genome, including polymorphic sites. Allele-specific primers and probes hybridizing to these sites and to regions flanking these sites are also provided. This invention further provides methods of analyzing a nucleic acid from an individual or a group of individuals. The nucleic acids, primers, and probes are useful for applications including forensics, paternity testing, medicine, e.g., the correlation of polymorphisms with phenotypic traits, and genetic analysis, e.g., genetic mapping of such phenotypic traits.

EP 1 262 565 A2

SEQUENCE LISTING

<110> Pfizer Products Inc.

<120> GENETIC POLYMORPHISMS IN THE HUMAN NEUROKININ 1 RECEPTOR GENE AND THEIR USES IN DIAGNOSIS AND TREATMENT OF DISEASES

<130> PC10461AGPR

<150> 60/293,425

<151> 2001-05-25

<160> 48

<170> PatentIn version 3.1

<210> 1
<211> 10
<212> DNA
<213> Homo sapiens

<400> 1
cactcagctg                                                              10

<210> 2
<211> 10
<212> DNA
<213> Homo sapiens

<400> 2
cactctgctg                                                              10

<210> 3
<211> 10
<212> DNA
<213> Homo sapiens

<400> 3
gcggaccgca 10

<210> 4

<211> 10

<212> DNA

<213> Homo sapiens

<400> 4
gcggacggca 10

<210> 5

<211> 11

<212> DNA

<213> Homo sapiens

<400> 5
tcccacggca a 11

<210> 6

<211> 11

<212> DNA

<213> Homo sapiens

<400> 6
tcccactgca a 11

<210> 7

<211> 12

<212> DNA

<213> Homo sapiens

<400> 7
gcccagaaaa ag 12

<210> 8

<211> 12

```
<212>   DNA

<213>   Homo sapiens


<400>   8                                                              12
gcccataaaa ag


<210>   9

<211>   11

<212>   DNA

<213>   Homo sapiens



<400>   9                                                              11
cagccacagg a


<210>   10

<211>   11

<212>   DNA

<213>   Homo sapiens



<400>   10                                                             11
cagcgacagg a


<210>   11

<211>   10

<212>   DNA

<213>   Homo sapiens



<400>   11                                                             10
taccgcctag


<210>   12

<211>   10

<212>   DNA

<213>   Homo sapiens



<400>   12                                                             10
taccgcttag
```

<210> 13

<211> 11

<212> DNA

<213> Homo sapiens

<400> 13
caccctcgtc c　　　　　　　　　　　　　　　　　　　　　　　11

<210> 14

<211> 11

<212> DNA

<213> Homo sapiens

<400> 14
caccctcatc c　　　　　　　　　　　　　　　　　　　　　　　11

<210> 15

<211> 11

<212> DNA

<213> Homo sapiens

<400> 15
agtatgggtt a　　　　　　　　　　　　　　　　　　　　　　　11

<210> 16

<211> 11

<212> DNA

<213> Homo sapiens

<400> 16
agtatggctt a　　　　　　　　　　　　　　　　　　　　　　　11

<210> 17

<211> 13

<212> DNA

<213> Homo sapiens

<400> 17

```
tgctcgctct cca                                                    13


<210>  18

<211>  13

<212>  DNA

<213>  Homo sapiens


<400>  18
tgctcgcact cca                                                    13


<210>  19

<211>  20

<212>  DNA

<213>  Homo sapiens


<400>  19
ccagccttaa agcacttccc                                             20


<210>  20

<211>  20

<212>  DNA

<213>  Artificial


<400>  20
ttccaagacg ttgtgtgtcc                                             20


<210>  21

<211>  19

<212>  DNA

<213>  Artificial


<400>  21
agcagatcag caacaaccg                                              19


<210>  22

<211>  20

<212>  DNA

<213>  Artificial
```

<400> 22
tcacgagaga ttccagctcc                                                    20

<210> 23

<211> 20

<212> DNA

<213> Artificial


<400> 23
tgaccagatc cctgaattgg                                                    20

<210> 24

<211> 20

<212> DNA

<213> Artificial


<400> 24
acctggctag ctgattcacc                                                    20

<210> 25

<211> 20

<212> DNA

<213> Artificial


<400> 25
ttctgacaga actgatgcgc                                                    20

<210> 26

<211> 20

<212> DNA

<213> Artificial


<400> 26
aggatttggg aaacagctgg                                                    20

<210> 27

<211> 22

<212> DNA

<213> Artificial

<400> 27
gtttcctaaa cacagcctca gg          22

<210> 28

<211> 20

<212> DNA

<213> Artificial

<400> 28
tttgctgctt gctacttgcc          20

<210> 29

<211> 20

<212> DNA

<213> Artificial

<400> 29
acttccaagc cacctactgc          20

<210> 30

<211> 20

<212> DNA

<213> Artificial

<400> 30
tctggagctt cgtatccagc          20

<210> 31

<211> 20

<212> DNA

<213> Artificial

<400> 31
tccagaatcc tggccatagg          20

```
<210>  32
<211>  18
<212>  DNA
<213>  Artificial


<400>  32
ttgaaagctg aactggcg                                                    18


<210>  33
<211>  21
<212>  DNA
<213>  Artificial


<400>  33
aaagtggagg aattcagagc c                                                21


<210>  34
<211>  20
<212>  DNA
<213>  Artificial


<400>  34
cacatcacta ccacgttgcc                                                  20


<210>  35
<211>  19
<212>  DNA
<213>  Artificial


<400>  35
ccgagagatg ctttgagcg                                                   19


<210>  36
<211>  19
<212>  DNA
<213>  Artificial


<400>  36
```

```
aaagccaccg cctacatcc                                              19


<210>   37

<211>   22

<212>   DNA

<213>   Artificial


<400>   37
acagatagta gggctttacc gc                                          22


<210>   38

<211>   22

<212>   DNA

<213>   Artificial


<400>   38
agttccaaac ctcacagcaa tg                                          22


<210>   39

<211>   23

<212>   DNA

<213>   Artificial


<400>   39
ttgttctttc ttctctgttc cag                                         23


<210>   40

<211>   20

<212>   DNA

<213>   Artificial


<400>   40
accatcgttt ctttggcatg                                             20


<210>   41

<211>   23

<212>   DNA

<213>   Artificial
```

<400> 41
gacctttcct gtttaccttg ctg                                             23

<210> 42

<211> 19

<212> DNA

<213> Artificial


<400> 42
ctcacaagtg tgccatccc                                                  19


<210> 43

<211> 20

<212> DNA

<213> Artificial


<400> 43
cacctgtctc accctcttgc                                                 20


<210> 44

<211> 20

<212> DNA

<213> Artificial


<400> 44
tgtagatggt cttgtggccc                                                 20


<210> 45

<211> 19

<212> DNA

<213> Artificial


<400> 45
cacctcttca tctgctcgc                                                  19


<210> 46

<211> 20

```
<212>  DNA

<213>  Artificial


<400>  46
gaatttccat gcatgaaggg                                          20


<210>  47

<211>  20

<212>  DNA

<213>  Artificial


<400>  47
tccaatgtgc tctcctaggc                                          20


<210>  48

<211>  20

<212>  DNA

<213>  Artificial


<400>  48
atcctgaaat gagcactcgc                                          20
```

**Description**

FIELD OF THE INVENTION

This invention provides polymorphisms in the human neurokinin 1 receptor (TACR1) locus, nucleic acid segments that include such polymorphisms or serve as probes for such polymorphisms, and methods of analyzing a nucleic acid by determining it's polymorphic form.

BACKGROUND OF THE INVENTION

The genomes of all organisms undergo spontaneous mutation in the course of their continuing evolution, generating variant forms of progenitor sequences (see, e.g., Gusella, *Ann. Rev. Biochem. 55,* 831-54 (1986)). A variant form may be neutral or confer an evolutionary advantage or disadvantage relative to a progenitor sequence. In some instances, a variant form confers a lethal disadvantage. In other instances, a variant form confers an evolutionary advantage to the species and is eventually incorporated into the deoxyribonucleic acid (DNA) of many or most members of the species and effectively becomes the progenitor sequence. In many instances, both the progenitor sequence and the variant form(s) survive and co-exist in a species population. The coexistence of multiple forms of a sequence gives rise to polymorphisms.

Several different types of polymorphisms have been reported. A restriction fragment length polymorphism (RFLP) is a variation in a DNA sequence that alters the length of a restriction fragment (see, e.g., Botstein et al., *Am. J. Hum. Genet.,* 32:314-31 (1980)). The RFLP may create or delete a restriction site, thus changing the length of the restriction fragment. RFLPs have been widely used in human and animal genetic analyses (see, e.g., WO 90/13668; W0 90/11369; Donis-Keller, *Cell,* 51:319-37 (1987); and Lander et al., *Genetics,* 121:85-99 (1989)). Where a heritable trait can be linked to a particular RFLP, the presence of such RFLP can be used to predict the likelihood that the phenotypic trait will be expressed.

Other polymorphisms take the form of short tandem repeats (STRs), e.g., tandem di-, tri-, and tetra-nucleotide repeated motifs. These tandem repeats are also referred to as variable number tandem repeat (VNTR) polymorphisms. VNTRs have been used in identity and paternity analyses as well as in a large number of genetic mapping studies (see, e.g., US 5,075,217; Armour et al., *FEBS Lett.,* 307:113-15 (1992); Horn et al., W0 91/14003; and Jeffreys, EP 370,719).

Other polymorphisms take the form of single nucleotide variations between individuals of the same species. Such polymorphisms are far more frequent than RFLPs, STRs and VNTRs. Some single nucleotide polymorphisms (SNPs) occur in protein-coding sequences, in which case, one of the polymorphic forms may give rise to the expression of a defective or otherwise variant protein, and potentially a genetic disease. Examples of genes in which polymorphisms within coding sequences give rise to genetic disease include β-globin (sickle cell anemia) and CFTR (cystic fibrosis). SNPs also occur in noncoding regions and these SNPs may result in defective protein expression (e.g., as a result of defective splicing). Some SNPs have no phenotypic effects.

SNPs can be used in the same manner as RFLPs and VNTRs but offer several advantages. SNPs occur with greater frequency and are spaced more uniformly throughout the genome than other polymorphisms. The greater frequency and uniformity of SNPs mean that there is a greater probability that such a SNP will be found in close proximity to a genetic locus of interest than would be the case for other polymorphisms. Also, the different forms of characterized SNPs are often easier to distinguish than other polymorphisms (e.g., by use of assays employing allele-specific hybridization probes or primers).

Despite the increased amount of nucleotide sequence data being generated in recent years, only a minute proportion of the total repository of polymorphisms in humans and other organisms has been identified. The paucity of polymorphisms hitherto identified is due to, in substantial part, the large amount of work required for their detection by conventional methods. For example, a conventional approach to identifying polymorphisms sequences the same stretch of oligonucleotides in a population of individuals by direct-sequencing, e.g., dideoxy sequencing. In this type of approach, the amount of work increases in proportion to both the length of the sequence and the number of individuals in a population and, as those skilled in the art will understand, becomes impractical for large stretches of DNA or large numbers of subjects.

WO 00/06768 discloses genetic polymorphisms in the human neurokinin 1 receptor gene. The entirety of that disclosure is hereby incorporated by reference herein. However, none of the polymorphisms of the present invention are disclosed in that document.

The reader is referred to the following publications for background information: *Primary structure and gene organization of human substance P and neuromedin K receptors,* Takahashi et al., *Eur. J. Biochem.,* 204:1025-33 (1992); *Differential activation of intracellular effector by two isoforms of* the *human neurokinin-1 receptor,* Fong et al., *Molecular Pharmacology,* 41:24-30 (1992); *Human Substance P receptor (NK-1): organisation of the gene, chromo-*

*some localization, and functional expression of cDNA clones,* Gerard et al., *Biochemistry,* 30:10640-46 (1991); *Isolation and characterization of the human lung NK-1 receptor cDNA*, Hopkins et al., *Bioc. Biop. Res. Comm.,* 180:1110-17 (1991); *Mutational analysis of neurokinin receptor function,* Fong et al., *Can. J. Physiol. Pharmacol.,* 73:860-65 (1995); Structure *and function of G protein-coupled receptors,* Strader et al., *Ann. Rev. Biochem.,* 63:101-32 (1994); *The evolution and structure of aminergic G protein-coupled receptors,* Donnelly et al., *Receptors and Channels,* 2:61-78 (1994).

TACR1 polypeptide is known to exist in 2 isoforms, which are possibly alternatively spliced variants of a single TACR1 gene, see Fong, *supra.* A cDNA encoding TACR1 has been published in International patent application WO 92/16547, Children's Medical Center; and in European patent application EP 510,878, Merck.

The complete genomic sequence of TACR1 is known with regions described in Genbank: Exon 1, Accession number X 65177, 2472 bp; Exon 2, Accession number X65178, 594 bp; Exon 3, Accession number X65179, 373 bp; Exon 4, Accession number XX65180, 371 bp; Exon 5, Accession number X65181, 3929 bp. Apart from Accession Number X 65177, all positions herein relate to the positions indicated therein unless stated otherwise or apparent from the context. Smith et al. (WO 00/06768) disclose that part of the sequence presented in EMBL Accession Number X65177 is incorrect. Sequencing of genomic PCR products by Smith et al. showed that the nucleotide sequence from positions 262-758 of EMBL Accession Number X65177 is incorrect and a Blast search they performed showed that this erroneous sequence actually corresponds to positions 1231-1729 of EMBL Accession Number U37688 which encodes a gene similar to the human c-myc proto-oncogene. None of the specific polymorphisms identified herein however, fall within this erroneous sequence.

One approach made available by the present invention is to use knowledge of the specific polymorphisms of the invention to help identify patients most suited to therapy with particular pharmaceutical agents (this is often termed "pharmacogenomics"). Pharmacogenetics can also be used in pharmaceutical research to assist the drug selection process. Polymorphisms are used in mapping the human genome and to elucidate the genetic component of diseases. The reader is directed to the following references for background details on pharmacogenetics and other uses of polymorphism detection: Linder et al., *Clinical Chemistry,* 43:254 (1997); Marshall, *Nature Biotechnology,* 15:1249 (1997); International Patent Application WO 97/40462, Spectra Biomedical; and Schafer et al., *Nature Biotechnology,* 16:33 (1998).

A haplotype is a set of alleles found at linked polymorphic sites (such as within a gene) on a single (paternal or maternal) chromosome. If recombination within the gene is random, there may be as many as $2^n$ haplotypes, where 2 is the number of alleles at each SNP, and n is the number of SNPs. One approach to identifying mutations or polymorphisms which are correlated with clinical response is to carry out an association study using all the haplotypes that can be identified in the population of interest. The frequency of each haplotype is limited by the frequency of its rarest allele, so that SNPs with low frequency alleles are particularly useful as markers of low frequency haplotypes. As particular mutations or polymorphisms associated with certain clinical features, such as adverse or abnormal events, are likely to be of low frequency within the population, low frequency SNPs may be particularly useful in identifying these mutations (for examples see: De Stefano et al., *Ann. Hum.* Genet., 62:481-90 (1998); and Keightley et al., *Blood,* 93:4277-83 (1999)). Clinical trials have shown that patient response to treatment with pharmaceuticals is often heterogeneous. Thus, there is a need for improved approaches to pharmaceutical agent design and therapy.

## SUMMARY OF THE INVENTION

This invention relates to polymorphisms in the human neurokinin 1 receptor (TACR1) gene. The invention also relates to methods and materials for analysing allelic variation in the TACR1 gene and to the use of said polymorphisms in the diagnosis and treatment of TACR1 ligand mediated diseases, such as asthma.

The present invention involves SNPs in the gene for the human TACR1 protein. This invention provides nucleic acid segments of between 10 and 100 bases selected from SEQ ID NO:1 (the published sequence of TACR1), including at least one of the polymorphic sites as shown in TABLE 1. Complements of these segments are also provided by this invention. The segments can be DNA or RNA, and can be double- or single-stranded. Some segments are 10-20 or 10-50 bases in length. Preferred segments include a biallelic polymorphic site. The base occupying the polymorphic site in the segments can be the reference base or an alternative base (TABLE 1, column 3).

The invention further provides allele-specific oligonucleotides that hybridize to a segment of a fragment shown in TABLE 1, column 3, or its complement. These oligonucleotides can be probes or primers. Also provided are isolated nucleic acids comprising a sequence shown in TABLE 1, column 3, or the complement thereto, in which the polymorphic site within the sequence is occupied by a base other than the reference base shown in TABLE 1, column 3.

The invention further provides methods of analyzing a nucleic acid from a human subject. The novel methods determine which base is present at any one of the polymorphic sites shown in TABLE 1. Optionally, a set of bases occupying a set of the polymorphic sites shown in TABLE 1 is determined. These types of analyses can be performed on a plurality of subjects who are tested for the presence of a disease phenotype. The presence or absence of disease phenotype can then be correlated with a base or set of bases present at the polymorphic sites in the subjects tested.

DESCRIPTION OF THE FIGURE

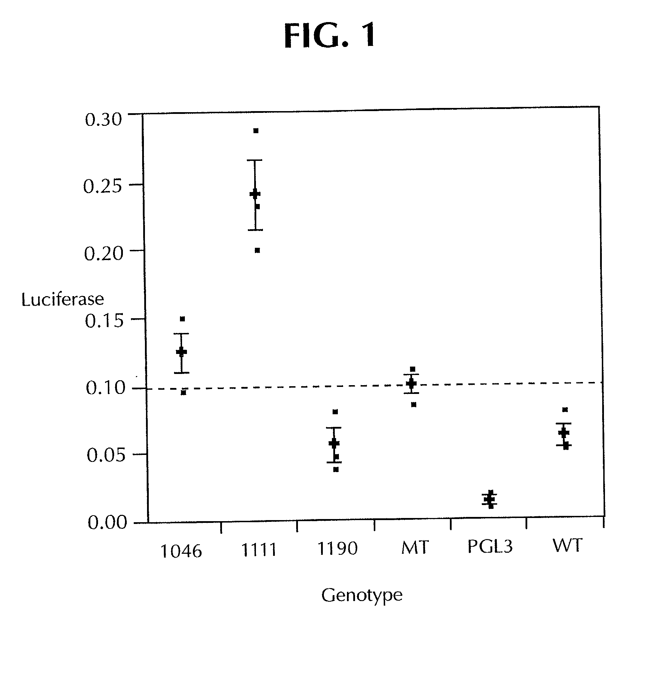

Figure 1 - A graphical presentation of promoter variation and transcriptional activity for a select group of polymorphic variations. In the graph, WT refers to the consensus sequence as defined in accession #X65177 with A at position 1046, C at position 1111, and G at position 1190. MT refers to a variant sequence compared to accession #X65177 with T at position 1046, G at position 1111, and T at position 1190. 1046 refers to another variant sequence with T at position 1046. 1111 refers to yet another variant sequence with G at position 1111. 1190 refers to yet another variant sequence with T at position 1190. G at position 1111 or T at position 1190 results in 4- and 2-fold increases in transcriptional activity, respectively, when compared to the consensus sequence from X65177 (p=.0001).

DEFINITIONS

An oligonucleotide, as the case may be, can be DNA or RNA, and single- or double-stranded. Oligonucleotides can be composed of naturally occurring or synthetic bases, but in either case are more commonly prepared by synthetic means. Preferred oligonucleotides of the invention include segments of DNA, or their complements, including any one of the polymorphic sites shown in TABLE 1. The segments are usually between 5 and 100 bases in length, and often between 5-10, 5-20, 10-20, 10-50, 20-50, or 20-100 bases in length. The polymorphic site can occur within any position of the segment. The segments can be from any of the allelic forms of DNA shown in TABLE 1.

Hybridization probes are oligonucleotides capable of binding in a base-specific manner to a complementary strand of nucleic acid. Such probes include peptide nucleic acids (see, e.g., Nielsen et al., *Science,* 254:1497-500 (1991)), as well as other nucleic acid forms.

A primer is a single-stranded oligonucleotide capable of acting as a point of initiation for template-directed DNA synthesis under suitable conditions (e.g., buffer and temperature), in the presence of the four different nucleoside triphosphates and an agent for polymerization, such as, for example, DNA or RNA polymerase or reverse transcriptase. The length of the primer, in any given case, depends on, for example, the intended use of the primer, and generally ranges from 15 to 30 nucleotides. A primer need not reflect the exact sequence of the template but must be sufficiently complementary to hybridize with such template. The primer site is the area of the template to which a primer hybridizes. The primer pair is a set of primers including a 5' upstream primer that hybridizes with the 5' end of the sequence to be amplified and a 3' downstream primer that hybridizes with the complement of the 3' end of the sequence to be amplified.

Linkage describes the tendency of genes, alleles, loci, or genetic markers to be inherited together as a result of their location on the same chromosome, and can be measured by percent recombination between the two genes, alleles, loci, or genetic markers.

Polymorphism refers to the occurrence of two or more genetically determined alternative sequences or alleles in a population. A polymorphic marker or site is the locus at which divergence occurs. Preferred markers have at least two alleles, each occurring at frequency of greater than about 1 percent (%), and more preferably greater than about 10%, within a selected population. A polymorphic locus may be as small as one base pair. Polymorphic markers include RFLPs, VNTRs, hypervariable regions, minisatellites, dinucleotide repeats, trinucleotide repeats, tetranucleotide repeats, simple sequence repeats, insertion elements (such as, for example, Alu), and, of course, SNPs. The first identified allelic form is arbitrarily designated the reference form while subsequently identified allelic forms are designated as alternative or variant alleles. The allelic form occurring most frequently in a selected population is often referred to as the wildtype form. Diploid organisms may be homozygous or heterozygous for allelic forms. A biallelic polymorphism has two forms.

A SNP occurs at a polymorphic site occupied by a single nucleotide, which is the site of variation between allelic sequences. This site of variation is usually both preceded and followed by highly conserved sequences, e.g., sequences that vary in less than 1/100 or 1/1000 members of the populations, of the given allele.

A SNP usually arises due to the substitution of one nucleotide for another at the polymorphic site. These substitutions include both transitions (i.e., the replacement of one purine by another purine or one pyrimidine by another pyrimidine) and transversions (i.e., the replacement of a purine by a pyrimidine, or vice versa). SNPs can also arise from either a deletion of a nucleotide or from an insertion of a nucleotide relative to a reference allele.

Hybridizations, e.g., allele-specific probe hybridizations, are generally performed under stringent conditions. For example, conditions where the salt concentration is no more than about 1 molar (M) and the temperature is at least about 25°C, e.g., 750 millimolar (mM) sodium chloride (NaCl), 50 mM sodium phosphate (NaPhosphate), and 5 mM ethylenediaminetetraacetic acid (EDTA), at pH 7.4 (5X SSPE) and a temperature of from about 25 °C to about 30°C.

An isolated nucleic acid is an object species that is the predominant species present (*i.e.*, on a molar basis it is more abundant than any other individual species in the composition). Preferably, an isolated nucleic acid comprises at least about 50, 80, or 90% (on a molar basis) of all macromolecular species present. More preferably, the object species is purified to essential homogeneity (contaminant species cannot be detected in the composition by conventional detection methods).

DETAILED DESCRIPTION OF THE INVENTION

I. NOVEL POLYMORPHISMS OF THIS INVENTION

The novel polymorphisms of this invention are listed in TABLE 1. The first column lists the genomic regions where the SNP lies. The second and third columns of TABLE 1 list the positions at which the polymorphism occurs with respect to transcriptional start site and Genbank reference sequence, respectively. These fragments are all human genomic fragments. Also included within the scope of this invention are all analogous fragments of other species to such human genomic fragments of TABLE 1. The fourth column of TABLE 1 lists the polymorphic site within the fragment. The base occupying the polymorphic site in the sequence in the database is bolded. This base is arbitrarily designated the reference, or prototypical form, but is not necessarily the most frequently occurring form. The fifth column lists the amino acid at the consensus site if the polymorphism resides in the coding region. The sixth column list the frequency of the variant in a population of 29-32 subjects, with the number showing the frequency of the amino acid in parentheses.

TABLE 1

| Region | Position* | Position in Reference Sequence | Sequence | A.Acid | Frequency |
|---|---|---|---|---|---|
| 5' region | -320 | 1046 in ×65177 | CACTCAGCTG (SEQ ID NO:1) CACTCTGCTG (SEQ ID NO:2) | none | 0.86 (A) |
| 5' region | -255 | 1111 in X65177 | GCGGACCGCA (SEQ ID NO:3) GCGGACGGCA (SEQ ID NO:4) | none | 0.92 (C) |
| 5' region | -176 | 1190 in X65177 | TCCCACGGCAA (SEQ ID NO:5) TCCCACTGCAA (SEQ ID NO:6) | none | 0.94 (G) |
| 5' UTR | 465 | 1831 in X65177 | GCCCAGAAAAAG (SEQ ID NO:7) GCCCATAAAAAG (SEQ ID NO:8) | none | NA |
| 5' UTR | 524 | 1890 in X65177 | CAGCCACAGGA (SEQ ID NO:9) CAGCGACAGGA (SEQ ID NO:10) | none | NA |
| 5' UTR | 600 | 1943 in X65177 | TACCGCCTAG (SEQ ID NO:11) TACCGCTTAG (SEQ ID NO:12) | none | 0.98 (C) |
| exon 5 | +1722 | 241 in X65181 | CACCCTCGTCC (SEQ ID NO:13) CACCCTCATCC (SEQ ID NO:14) | Ser to Ser | 0.98 (G) |
| 3'UTR | +1942 | 461 in X65181 | AGTATGGGTTA (SEQ ID NO:15) AGTATGGCTTA (SEQ ID NO:16) | none | 0.81 (G)† |

* Position based on start of primary transcription

TABLE 1   (continued)

| Region | Position* | Position in Reference Sequence | Sequence | A.Acid | Frequency |
|---|---|---|---|---|---|
| intron 4 | -6** | 33 in X65181 | TGCTCGCTCTCCA (SEQ ID NO:17) TGCTCGCACTCCA (SEQ ID NO:18) | none | 0.98 (T) |

* Position based on start of primary transcription

** Position based on relationship to start of Exon 5 † 3'UTR frequencies calculated based on SSCP genotyping assay, the other 5 changes were all calculated by sequencing.

## II. ANALYSIS of NOVEL POLYMORPHISMS

### A. PREPARATION of SAMPLES

Polymorphisms are detected in a target nucleic acid from a subject being analyzed. Any suitable biological sample can be used for assay of genomic DNA. Pure red blood cells are not suitable for use in such assays. Convenient suitable tissue samples include, for example, whole blood, semen, saliva, tears, urine, fecal material, sweat, buccal material, skin, and hair. As those skilled in the art will appreciate, for assays of cDNA or mRNA, the tissue sample must be obtained from an organ in which the target nucleic acid is expressed, e.g., the liver for a target nucleic acid of a cytochrome P450.

Many known methods such as, for example, those described below, require amplification of the DNA obtained from target samples, and such amplification is preferably accomplished by the polymerase chain reaction (PCR) (see, e.g., *PCR Technology: Principles and Applications for DNA Amplification* (ed. H.A. Erlich, Freeman Press, NY, NY, 1992); *PCR Protocols: A Guide to Methods and Applications* (eds. Innis, et al., Academic Press, San Diego, CA, 1990); Mattila et al., *Nucleic Acids Res.,* 19:4967 (1991); Eckert et al., *PCR Methods and Applications,* 1:17 (1991); *PCR* (eds. McPherson et al., IRL Press, Oxford); and U.S. Patent 4,683,202).

Other suitable amplification methods include the ligase chain reaction (LCR) (e.g., Wu and Wallace, *Genomics,* 4:560 (1989) and Landegren et al., *Science,* 241:1077 (1988)), transcription amplification (e.g., Kwoh et al., *Proc. Natl. Acad. Sci. USA,* 86:1173 (1989)), self-sustained sequence replication (e.g., Guatelli et al., *Proc. Nat. Acad. Sci. USA,* 87:1874 (1990)), and nucleic acid based sequence amplification (NABSA). The latter two amplification methods include isothermal reactions based on isothermal transcription, which produce both single-stranded RNA (ssRNA) and double-stranded DNA (dsDNA) as the amplification products in a ratio of about 30 or 100 to 1, respectively.

### B. DETECTION OF NOVEL POLYMORPHISMS IN TARGET DNA

There are two distinct types of analyses depending upon whether a given polymorphism has already been characterized. The first type of analysis is sometimes referred to as *de novo* characterization and compares target sequences in different subjects to identify points of variation, i.e., polymorphic sites. By analyzing a group of subjects representing the greatest ethnic diversity among human beings and the greatest breed and species variety of plants and animals, patterns characteristic of the most common alleles/haplotypes of a given locus can be identified, and the frequencies of such patterns in the population can be determined. Additional allelic frequencies can be determined for subpopulations characterized by criteria such as, for example, geography, race, or gender. This *de novo* characterization of the polymorphisms of this invention is described in the EXAMPLES section of this description. The second type of analysis determines which form(s) of a characterized polymorphism are present in the subjects being tested. Many suitable procedures exist and these are discussed herein.

### 1. ALLELE-SPECIFIC PROBES

The design and use of allele-specific probes for analyzing polymorphisms is known in the art, e.g., Saiki et al., *Nature,* 324:163-66 (1986), Dattagupta, EP 235,726, and Saiki, WO 89/11548. Allele-specific probes can be designed that hybridize to a segment of target DNA from one subject and not to the corresponding segment from another subject due to the presence of different polymorphic forms in their respective segments. Hybridization conditions should be suitably stringent so that there is a significant difference in hybridization intensity between alleles, preferably an essentially binary response, whereby the chosen probe hybridizes to only one of the alleles. Some probes are designed to hybridize to a segment of target DNA such that the polymorphic site aligns with a central position (e.g., in a 15 mer at the 7 position; in a 16 mer, at either the 8 or 9 position) of the probe. This particular design of probe achieves useful discrimination in hybridization between different allelic forms.

Allele-specific probes are often used in pairs, with one member of a pair showing a perfect match to a reference form of a target sequence and the other member showing a perfect match to a variant form. Several pairs of probes can then be immobilized on the same support for simultaneous analyses of multiple polymorphisms within the same target sequence.

## 2. TILING ARRAYS

Polymorphisms can also be identified by hybridization to nucleic acid arrays (see, e.g., WO 95/11995). One form of such arrays is described in the EXAMPLES section of this description in connection with de novo identification of polymorphisms. The same array, or a different array, can be used for analyses of characterized polymorphisms. The aforementioned WO 95/11995 also discloses subarrays that are optimized for the detection of variant forms of a pre-characterized polymorphism. Such subarrays contain probes designed to be complementary to a second reference sequence, which is an allelic variant of the first reference sequence. The second group of probes is designed by the same principles as described in the EXAMPLES in this description, except that the probes exhibit complementarity to the second reference sequence. The inclusion of a second group (or further groups) can be particularly useful for analyzing short subsequences of the primary reference sequence in which multiple mutations are predicted to occur within a short distance commensurate with the length of the probes (i.e., two or more mutations within 9 to 21 bases).

## 3. ALLELE-SPECIFIC PRIMERS

An allele-specific primer hybridizes to a site on a target DNA overlapping a polymorphism and only primes amplification of an allelic form to which the primer exhibits perfect complementarity (see, e.g., Gibbs, *Nucleic Acid Res.,* 17:2427-48 (1989)). This primer is used in conjunction with a second primer which hybridizes at a distal site. Amplification proceeds from these two primers leading to a detectable product which signifies that the particular allelic form is present. A control is generally performed with a second pair of primers, one of which shows a single base mismatch at the polymorphic site and the other of which exhibits perfect complementarity to the distal site. This single-base mismatch prevents amplification and, as such, no detectable product is formed. Most preferably, the mismatch is included in the 3'-most position of the oligonucleotide aligned with the polymorphism, i.e., the 3'-most position is the position most destabilizing to elongation from the primer (see, e.g., WO 93/22456).

## 4. DIRECT-SEQUENCING

The direct analysis of the sequence of polymorphisms of this invention can be accomplished by using either the dideoxy chain termination method or the Maxam Gilbert method (e.g., Sambrook et al., *Molecular Cloning, A Laboratory Manual* (2nd Ed., CSHP, New York 1989) and Zyskind et al., *Recombinant DNA Laboratory Manual* (Acad. Press, 1988)).

## 5. DENATURING GRADIENT GEL ELECTROPHORESIS

Amplification products generated using PCR can be analyzed by the use of denaturing gradient gel electrophoresis. Different alleles can be identified based on the different sequence-dependent melting properties and electrophoretic migration of DNA in solution (see, e.g., Erlich, ed., *PCR Technology, Principles and Applications for DNA Amplification,* (W.H. Freeman and Co, New York, 1992), Chapter 7)).

## 6. SINGLE-STRAND CONFORMATION POLYMORPHISM ANALYSIS

Alleles of target sequences can be differentiated using single-strand conformation polymorphism analysis, which identifies base differences by alteration in electrophoretic migration of single-stranded PCR products (see, e.g., Orita et al., *Proc. Nat. Acad. Sci.,* 86:2766-70 (1989)). Amplified PCR products can be generated as described above, and heated or otherwise denatured, to form single-stranded amplification products. Single-stranded nucleic acids may refold or form secondary structures which are partially dependent on the base sequence. The different electrophoretic mobilities of the single-stranded amplification products can be related to base-sequence differences between alleles of the target sequences.

Additional methods for the detecting the polymorphisms described in TABLE 1, include, without limitation, fluorescence polarization, mass spectrometry, 5' exonuclease assays (TaqMan™, Roche Molecular Systems, Inc. Corp., Somerville, NJ), restriction fragment length polymorphism, fluorescence resonance energy transfer (FRET), any methodology that reads the product of a primer-extension reaction and hybridization-based technologies. Such methods are well known to those of the skill in the art and are described, e.g., in Ausujel, et al., *Current Protocols in Molecular Biology,* John Wiley Sons, New York, NY (1999) and Sambrook et al., *Molecular Cloning: A Laboratory Manual,* 2d Ed., Cold Spring Harbor laboratory Press, Plainview, NY (1989).

III. METHODS OF USE

The presence of polymorphic form(s) in a subject at one or more polymorphic sites is useful information for, e.g., forensics, paternity testing, correlating of polymorphisms with phenotypic traits, and genetic mapping of phenotypic traits.

A. FORENSICS

Determination of which polymorphic forms occupy a set of polymorphic sites in an individual identifies a set of polymorphic forms that distinguishes the individual (see, e.g., National Research Council, *The Evaluation of Forensic DNA Evidence* (Eds. Pollard et al., National Academy Press, DC, 1996)). Increasing the number of sites analyzed lessens the probability that the set of polymorphic forms in one individual is the same as that in an unrelated individual. Preferably, where multiple sites are analyzed, the sites are unlinked. Thus, polymorphisms of this invention are often used in conjunction with polymorphisms in distal genes. Preferred polymorphisms for use in forensics are biallelic because the population frequencies of two polymorphic forms can usually be determined with greater accuracy than those of multiple polymorphic forms at multi-allelic loci.

As discussed above, the capacity to identify a distinguishing or unique set of forensic markers in an individual is useful for forensic analysis. For example, one can determine whether a blood sample from a suspect matches a blood or other tissue sample from a crime scene by determining whether the set of polymorphic forms occupying selected polymorphic sites is the same in the sample taken from the suspect and the sample taken from the crime scene. Where the set of polymorphic markers taken from the crime scene does not match the sample taken from the suspect, it can be concluded (barring experimental error) that the suspect is not the source of the sample taken from the crime scene. Where the set of markers does match, one can conclude that the DNA from the suspect is consistent with that found at the crime scene. Where frequencies of the polymorphic forms at the loci tested have been determined (e.g., by analysis of a suitable population of individuals), a statistical analysis can be performed to determine the probability that such a match of suspect and crime scene would occur merely by chance.

p(ID) is the probability that two random individuals have the same polymorphic or allelic form at a given polymorphic site. In biallelic loci, four genotypes are possible: AA, AB, BA, and BB. Where alleles A and B occur in a haploid genome of the organism with frequencies x and y, the probability of each genotype in a diploid organism, as disclosed in WO 95/12607, is:

Homozygote: $p(AA) = x^2$.

Homozygote: $p(BB) = y^2 = (1-x)^2$.

Single Heterozygote: $p(AB) = p(BA) = xy = x(1-x)$.

Both Heterozygotes: $p(AB+BA) = 2xy = 2x(1-x)$.

The probability of identity at one locus (i.e., the probability that two individuals, picked at random from a population will have identical polymorphic forms at a given locus) is given by the equation:

$$p(ID) = (x^2)^2 + (2xy)^2 + (y^2)^2.$$

These calculations can be extended for any number of polymorphic forms at a given locus. For example, the probability of identity p(ID) for a 3-allele system where the alleles have the frequencies in the population of x, y and z, respectively, is equal to the sum of the squares of the genotype frequencies:

$$p(ID) = x^4 + (2xy)^2 + (2yz)^2 + (2xz)^2 + z^4 + y^4.$$

In a locus of n alleles, the appropriate binomial expansion is used to calculate p(ID) and p(exc).

The cumulative probability of identity (cum p(ID)) for each of multiple unlinked loci is determined by multiplying the probabilities provided by each locus.

$$cum\ p(ID) = p(ID1)p(ID2)p(ID3)....\ p(IDn).$$

The cumulative probability of non-identity for w loci (i.e., the probability that two random individuals will be different at 1 or more loci) is given by the equation:

$$cum\ p(nonID) = 1\text{-}cum\ p(ID).$$

If several polymorphic loci are tested, the cumulative probability of non-identity for random individuals becomes very high (e.g., one in a billion). Such probabilities can be taken into account together with other evidence in determining the guilt or innocence of the suspect.

B. PATERNITY TESTING

The object of paternity testing is usually to determine whether a particular male is the father of a given child. In most cases, the mother of the child is known and thus, the mother's contribution to the child's genotype can be traced. Hence, paternity testing investigates whether the part of the child's genotype not attributable to the mother is consistent with that of the alleged father. Paternity testing can be performed by analyzing the sets of polymorphisms in both the alleged father and in his alleged child.
Where the set of polymorphisms in the child not attributable to the child's mother does not match the set of the alleged father, it can be concluded (barring experimental error) that the alleged father is not the child's father. Where the set of polymorphisms in the child not attributable to the child's mother does match the set of polymorphisms of the alleged father, a statistical calculation can be performed to determine the probability of a coincidental match.
The probability of parentage exclusion (representing the probability that a random male will have a polymorphic form at a given polymorphic site that makes him incompatible as the father) is given by the equation disclosed in WO 95/12607 as:

$$p(exc) = xy(l\text{-}xy)$$

where x and y are the population frequencies of alleles A and B of a biallelic polymorphic site (at a triallelic site p(exc) = xy(1-xy) + yz(1- yz) + xz(l-xz)+ 3xyz(l-xyz), where x, y, and z are the respective population frequencies of alleles A, B, and C).
The probability of non-exclusion is:

$$p(non\text{-}exc) = 1\text{-}p(exc).$$

The cumulative probability of non-exclusion (representing the value obtained when n loci are used) is thus:

$$cum\ p(non\text{-}exc) = p(non\text{-}exc1)p(non\text{-}exc2)p(non\text{-}exc3)....\ p(non\text{-}excn).$$

The cumulative probability of exclusion for z loci (representing the probability that a random male will be excluded):

$$cum\ p(exc) = 1 - cum\ p(non\text{-}exc).$$

Where several polymorphic loci are included in the analysis, the cumulative probability of exclusion of a random male is very high. This probability can be taken into account in assessing the probability of an alleged father whose polymorphic marker set matches the child's polymorphic marker set attributable to the child's father.

## C. CORRELATION OF NOVEL POLYMORPHISMS WITH PHENOTYPIC TRAITS

The polymorphisms of this invention may contribute to the phenotype of an organism in different ways. As discussed above, some polymorphisms occur within a protein coding sequence and contribute to phenotype by affecting protein structure. The effect of such a change to the structure of a protein may be neutral, beneficial, or detrimental, or both beneficial and detrimental. For instance, a heterozygous sickle cell mutation confers resistance to malaria, but a homozygous sickle cell mutation is usually lethal. Other polymorphisms occur in noncoding regions and exert phenotypic effects indirectly via influence on, e.g., replication, transcription, or translation. Moreover, a single polymorphism may affect more than one phenotypic trait. Likewise, a single phenotypic trait may be affected by polymorphisms in different genes. Further, some polymorphisms predispose an individual to a distinct mutation that is causally related to a certain phenotype.

Phenotypic traits include diseases that have known (but hitherto unmapped) genetic components (e.g., agammaglobulinemia, diabetes insipidus, Lesch-Nyhan syndrome, muscular dystrophy, Wiskott-Aldrich syndrome, Fabry's disease, familial hypercholesterolemia, polycystic kidney disease, hereditary spherocytosis, von Willebrand's disease, tuberous sclerosis, hereditary hemorrhagic telangiectasia, familial colonic polyposis, Ehlers-Danlos syndrome, osteogenesis imperfecta, acute intermittent porphyria, and the like). Phenotypic traits also include symptoms of, or susceptibility to, multifactorial diseases of which a component is, or may be, genetic, such as autoimmune diseases, inflammation, cancer, diseases of the nervous system, and infection by pathogenic microorganisms. Some examples of autoimmune diseases include rheumatoid arthritis, multiple sclerosis, diabetes (insulin-dependent and non-independent), systemic lupus erythematosus, and Graves disease. Some examples of cancers include cancers of the bladder, brain, breast, colon, esophagus, kidney, uterus, liver, lung, oral cavity, ovary, pancreas, prostate, skin, stomach, and leukemia. Phenotypic traits also include characteristics such as longevity, appearance (e.g., baldness, obesity), strength, speed, endurance, fertility, and susceptibility (or receptivity) to particular drugs or therapeutic treatments.

Correlation of polymorphisms with phenotypic traits is performed for a population of subjects who have been tested for the presence or absence of a phenotypic trait of interest and for polymorphic marker sets. To perform such analysis, the presence or absence of a set of polymorphisms (i.e., a polymorphic set) is determined for a set of the subjects, some of whom exhibit a particular trait, and some of whom do not. The alleles of each polymorphism of the set are then reviewed to determine whether the presence or absence of a particular allele is associated with the phenotypic trait of interest. Correlation can be performed by using standard statistical methods such as a κ-squared test and by noting any statistically significant correlations between the polymorphic form(s) and the phenotypic characteristics. For example, it might be found that the presence of allele A1 at polymorphism A correlates with heart disease. As a further example, it might be found that the combined presence of allele A1 at polymorphism A and allele B1 at polymorphism B correlates with increased milk production of, e.g., a farm animal.

These correlations are useful in several ways. For example, where a strong correlation exists between a set of one or more polymorphic forms and a disease for which treatment is available, detection of such polymorphic form set in a subject, i.e., a human being or an animal, may justify immediate administration of the treatment, or at least the institution of regular monitoring of the subject. In addition, detection of a polymorphic form correlated with a serious disease can assist with certain decision making. For example, where a couple is contemplating a family, and one or both has a polymorphic form correlated with a serious disease, the couple may take this into account when making their reproductive decisions. For instance, the female partner might elect to undergo *in vitro* fertilization to avoid the possibility of transmitting such a polymorphism from her husband to her offspring. Moreover, where a weaker yet statistically significant correlation exists between a polymorphic set and a human disease, immediate therapeutic intervention or monitoring may not be justified. Nevertheless, the patient can be motivated to begin simple life-style changes (e.g., diet, exercise, and the like) that can be accomplished at little monetary cost to the patient in exchange for potential benefits in reducing the risk of conditions to which the patient may have increased susceptibility by virtue of the variant alleles. Further, identification of a polymorphic set in a patient correlated with enhanced receptivity to one of several treatment regimes for a disease indicates that this treatment regime should be followed.

For animals and plants, correlations between characteristics and phenotype are useful for, e.g., breeding for desired characteristics. For example, Beitz et al., in US 5,292,639, disclose the use of bovine mitochondrial polymorphisms in a breeding program to improve milk production in cows.

## D. GENETIC MAPPING OF PHENOTYPIC TRAITS

The previous section described the identification of correlations between phenotypic traits and polymorphisms that directly or indirectly contribute to those traits. The present section describes the identification of a physical linkage between a genetic locus associated with a trait of interest and polymorphic markers that are not associated with that trait, but rather are in physical proximity with the genetic locus responsible for the trait and co-segregate with it. Such analysis is useful for, e.g., mapping a genetic locus associated with a phenotypic trait to a chromosomal position, and thereby cloning the gene(s) responsible for the trait (see, e.g., Lander et al., *Proc. Natl. Acad. Sci. (USA),* 83:7353-57 (1986); Lander et al., *Proc. Natl. Acad. Sci.* (USA), 84:2363-67 (1987); Donis-Keller et al., *Cell,* 51:319-37 (1987); and Lander et al., *Genetics,* 121:185-99 (1989)). Genes localized by linkage can be cloned by a process known as directional cloning (see, e.g., Wainwright, *Med. J. Australia,* 159:170-74 (1993); and Collins, *Nature Genetics,* 1:3-6 (1992)).

Linkage studies are generally performed on members of a family. Available members of the family are characterized for the presence or absence of a phenotypic trait and for a set of polymorphic markers. The distribution of polymorphic markers in an informative meiosis is then analyzed to determine which polymorphic markers co-segregate with a phenotypic trait (see, e.g., Kerem et al., *Science,* 245:1073-80 (1989); Monaco et al., *Nature,* 316:842 (1985); Yamoka et al., *Neurology,* 40:222-26 (1990); and Rossiter et al., *FASEB Journal,* 5:21-27 (1991)).

Linkage is analyzed by calculation of log of the odds (LOD) values. A LOD value is the relative likelihood of obtaining observed segregation data for a marker and a genetic locus when the two are located at a recombination fraction $\theta$, versus the situation in which the two are not linked, and thus segregating independently (see, e.g., Thompson & Thompson, *Genetics in Medicine* (5th ed., W.B. Saunders Company, Philadelphia, 1991); and Strachan, "Mapping the human genome" in *The Human Genome* (BIOS Scientific Publishers Ltd., Oxford), Chapter 4). A series of likelihood ratios are calculated at various $\theta$, ranging from $\theta = 0.0$ (coincident loci) to $\theta = 0.50$ (unlinked). Thus, the likelihood at a given value of $\theta$ is the probability of data where loci are linked at $\theta$ to the probability of data where loci are unlinked. The computed likelihoods are usually expressed as the $\log_{10}$ of this ratio (i.e., a LOD value). For example, a LOD value of 3 indicates 1000:1 odds against an apparent observed linkage being a coincidence. The use of logarithms allows data collected from different families to be combined by simple addition. Computer programs are available for the calculation of LOD scores for differing values of $\theta$ (e.g., LIPED or MLINK (see, e.g., Lathrop, *Proc. Nat. Acad. Sci. (USA)*, 81:3443-46 (1984)). For any particular LOD value, a recombination fraction may be determined using mathematical tables (see, e.g., Smith et al., *Mathematical tables for research workers in human genetics* (Churchill, London, 1961); and Smith, *Ann. Hum. Genet.*, 32:127-50 (1968)). The value of $\theta$ at which the LOD score is the highest is considered to be the best estimate of the recombination fraction.

Positive LOD values suggest that the two loci are linked, whereas negative LOD values suggest that linkage is less likely (at that value of $\theta$) than the possibility that the two loci are unlinked. By convention, a combined LOD value of +3 or greater (equivalent to greater than 1000:1 odds in favor of linkage) is considered definitive evidence that the two loci are linked. Similarly, by convention, a negative LOD value of -2 or less is taken as definitive evidence against linkage of the two loci being compared. Negative linkage data are useful in excluding a chromosome or a segment thereof from consideration. The search then focuses on the remaining non-excluded chromosomal locations.

IV. <u>KITS</u>

This invention further provides kits comprising at least one allele-specific oligonucleotide as described herein. Preferably, the kits contain one or more pairs of allele-specific oligonucleotides capable of hybridizing to different forms of a polymorphism. In some kits, the allele-specific oligonucleotides are provided immobilized to a substrate. For example, the same substrate can comprise allele-specific oligonucleotide probes for detecting any number of the polymorphisms shown in TABLE 1. Optional additional components of the kit include, for example, restriction enzymes, reverse-transcriptase or polymerase, the substrate nucleoside triphosphates, means used to label (for example, an avidin-enzyme conjugate and enzyme substrate and chromogen when the label is biotin), and the appropriate buffers for reverse transcription, PCR, or hybridization reactions. Generally, the kit also contains instructions for carrying out the methods.

<u>EXAMPLES</u>

The polymorphisms shown in TABLE 1 were identified by resequencing of target sequences from 29-32 unrelated individuals of diverse ethnic and geographic backgrounds by direct sequencing using dye terminator chemistry as described in the ABI protocol (Applied Biosystems, Foster City, CA). DNA was prepared from cultured lymphoblast cell lines following protocol I (Molecular Cloning: A Laboratory Manual, Sambrook, Fritsch, and Maniatis, 2d Edition, Cold Spring Harbor Press (1989)). Samples were extracted with phenol, then phenol/chloroform, and then chloroform rather than with three phenol extractions. The DNA was dissolved in deionised water. Templates were prepared by PCR using the oligonucleotide primers described in Table 2 below. The extension temperature was 72°C and denaturation temperature 94°C. Generally 50 ng of genomic DNA was used in each reaction and subjected to 35 cycles of PCR.

## Table 2

| Region | Position | primer ID * | sequence | ref. seq | ref. seq. pos. | amplicon |
|---|---|---|---|---|---|---|
| 5' UTR | 41+ | 35791-274 | CCAGCCTTAAAGCACTTCCC (SEQ ID NO:19) | X65177 | 41-60 | 41-389 |
| 5' UTR | 389- | 35791-275 | TTCCAAGACGTTGTGTGTCC (SEQ ID NO:20) | X65177 | 370-389 | |
| 5' UTR | 301+ | 35791-276 | AGCAGATCAGCAACAACCG (SEQ ID NO:21) | X65177 | 301-319 | 301-658 |
| 5' UTR | 658- | 35791-277 | TCACGAGAGATTCCAGCTCC (SEQ ID NO:22) | X65177 | 639-658 | |
| 5' UTR | 434+ | 35791-278 | TGACCAGATCCCTGAATTGG (SEQ ID NO:23) | X65177 | 434-450 | 434-802 |
| 5' UTR | 802- | 35791-279 | ACCTGGCTAGCTGATTCACC (SEQ ID NO:24) | X65177 | 783-802 | |
| 5' UTR | 678+ | 35791-280 | TTCTGACAGAACTGATGCGC (SEQ ID NO:25) | X65177 | 678-697 | 678-1063 |
| 5' UTR | 1063- | 35791-281 | AGGATTTGGGAAACAGCTGG (SEQ ID NO:26) | X65177 | 1044-1063 | |
| 5' UTR | 829+ | 35791-282 | GTTTCCTAAACACAGCCTCAGG (SEQ ID NO:27) | X65177 | 829-850 | 829-1209 |
| 5' UTR | 1209- | 35791-283 | TTTGCTGCTTGCTACTTGCC (SEQ ID NO:28) | X65177 | 1190-1209 | |
| 5' region | 1127+ | 35791-284 | ACTTCCAAGCCACCTACTGC (SEQ ID NO:29) | X65177 | 1127-1146 | 1127-1468 |
| 5' region | 1468- | 35791-285 | TCTGGAGCTTCGTATCCAGC (SEQ ID NO:30) | X65177 | 1449-1468 | |
| 5' region | 1463+ | 35791-286 | TCCAGAATCCTGGCCATAGG (SEQ ID NO:31) | X65177 | 1463-1482 | 1463-1815 |
| 5' region | 1816- | 35791-287 | TTGAAAGCTGAACTGGCG (SEQ ID NO:32) | X65177 | 1798-1815 | |
| 5' region | 1731+ | 35791-288 | AAAGTGGAGGAATTCAGAGCC (SEQ ID NO:33) | X65177 | 1731-1751 | 1731-2117 |
| 5' region | 2117- | 35791-289 | CACATCACTACCACGTTGCC (SEQ ID NO:34) | X65177 | 2098-2117 | |
| 5' UTR | 59+ | 35791-327 | CCGAGAGATGCTTTGAGCG (SEQ ID NO:35) | X65177 | 59-77 | 59-510 |
| 5' UTR | 492+ | 35791-328 | AAAGCCACCGCCTACATCC (SEQ ID NO:36) | X65177 | 492-510 | |
| Exon 1 | 1921+ | 35791-210 | ACAGATAGTAGGGCTTTACCGC (SEQ ID NO:37) | X65177 | 1921-1942 | 1921-2415 |
| Exon 1 | 2415- | 35791-211 | AGTTCCAAACCTCACAGCAATG (SEQ ID NO:38) | X65177 | 2394-2415 | |
| Exon 2 | 129+ | 35791-212 | TTGTTCTTTCTTCTCTGTTCCAGG (SEQ ID NO:39) | X65178 | 129-152 | 129-385 |
| Exon 2 | 385- | 35791-213 | ACCATCGTTTCTTTGGCATG (SEQ ID NO:40) | X65178 | 366-385 | |
| Exon 3 | 84+ | 35791-214 | GACCTTTCCTGTTTACCTTGCTG (SEQ ID NO:41) | X65179 | 84-106 | 84-337 |
| Exon 3 | 337- | 35791-215 | CTCACAAGTGTGCCATCCC (SEQ ID NO:42) | X65179 | 319-337 | |
| Exon 4 | 23+ | 35791-216 | CACCTGTCTCACCCTCTTGC (SEQ ID NO:43) | X65180 | 23-42 | 23-292 |
| Exon 4 | 292- | 35791-217 | TGTAGATGGTCTTGTGGCCC (SEQ ID NO:44) | X65180 | 273-292 | |
| Exon 5 | 14+ | 35791-218 | CACCTCTTCATCTGCTCGC (SEQ ID NO:45) | X65181 | 14-32 | 14-399 |
| Exon 5 | 399- | 35791-219 | GAATTTCCATGCATGAAGGG (SEQ ID NO:46) | X65181 | 380-399 | |
| 3'UTR | 1329+ | 35791-208 | TCCAATGTGCTCTCCTAGGC (SEQ ID NO:47) | X65181 | 314-333 | 314-659 |
| 3'UTR | 1674- | 35791-209 | ATCCTGAAATGAGCACTCGC (SEQ ID NO:48) | X65181 | 640-659 | |

12

In another embodiment, the invention comprises a method for predicting over-expression of the NK-1 receptor gene due to variation within the promoter region, specifically at positions 1046, 1111, and 1190 of accession # X65177. Specifically a T at position 1046, a G at position 1111, and a T at position 1190 are seen to affect expression (see Figure 1). The 5' region of the TACR1 gene comprised of nucleotides 1020-1458 (accession # X65177) was amplified from genomic DNA and cloned into the PGL-3 luciferase reporting construct per manufacturer's protocol (Promega Madison, WI). Site directed mutagenesis was performed to construct the vector containing all three variants according to the manufacturers protocol (Quickchange™ kit, Stratagene, La Jolla, CA)). Individual constructs were derived by amplifying the region from subjects known to harbor the variant allele at each respective position. Each clone was verified by direct sequencing using the protocol described above. Clones were transfected into the IM-9 lymphoblast cell line (#CCL-159, ATCC, Manassas, VA) using standard electroporation techniques. Transfection efficiency was normalized to a vector expressing a CMV-driven Green fluorescent protein (pEGFP, Clonetech, Palo Alto, CA). Luciferase values were read on a Dynex MLX fluorescence reader (Dynex Technologies, Middlesex, UK) and GFP levels were quantitated via FACS analysis using the FACSort cytometer according to manufacturers protocol (Becton-Dickinson, San Jose, CA).

It will be apparent to the person skilled in the art that there are a large number of analytical procedures which may be used to detect the presence or absence of variant nucleotides at one or more polymorphic positions of the invention. In general, the detection of allelic variation requires a mutation discrimination technique, optionally an amplification reaction and optionally a signal generation system. Table 3 lists a number of mutation detection techniques, some based on the polymerase chain reaction (PCR). These may be used in combination with a number of signal generation or detection systems, including FRET, fluorescence quenching, fluorescence polarisation (UK Patent No. 2,228,998 (Zeneca Limited)), chemiluminescence, electrochemiluminescence, Raman, radioactivity, calorimetric, hybridisation protection assay, mass spectrometry, SERRS (WO 97/05280 (University of Strathclyde)), and the like. Further amplification techniques include SSR, NASBA, LCR, SDA, b-DNA, and the like. Many current methods for the detection of allelic variation are reviewed by Nollau et al., *Clin. Chem.,* 43:1114-20 (1997); and in standard textbooks, for example "Laboratory Protocols for Mutation Detection", Ed. by U. Landegren, Oxford University Press (1996), and "PCR", 2d Edition, by Newton & Graham, BIOS Scientific Publishers Limited (1997).

Table 3 -

| Mutation Detection Techniques | |
|---|---|
| Technique Type | Examples |
| General | DNA sequencing, sequencing by hybridisation |
| Scanning | protein truncation test (PTT)*, single stranded conformation polymorphism (SSCP), denaturing gradient gel electrophoresis (DGGE), temperature gradient gel electrophoresis (TGGE), cleavase, heteroduplex analysis, chemical mismatch cleavage (CMC), enzymatic mismatch cleavage |
| Hybridisation Based | dot blots, multiplex allele-specific diagnostic assay (MASDA), Reverse dot blots, oligonucleotide arrays (e.g., DNA Chips), Taqman™ (see U.S. Patents 5,210,015 and 5,487,972 to Hofmann-La Roche), molecular beacons (see Tyagi et al., *Nature Biotechnology,* 14:303 (1996); WO 95/13399; Public Health Inst., New York) |
| Extension Based | allele refractory mutation scanning (ARMS™; also called allele specific amplification; as described in European patent EP-B-332,435 and U.S. Patent 5,595,890), ALEX™ (see European Patent EP-332,435-BI to Zeneca Limited), fluorescence polarization template-directed dye-terminator incorporation (FP-TDI; see Chen et al., Genome *Research,* 9:492-98 (1999)). |
| Incorporation Based | Mini-sequencing, arrayed primer extension (APEX™) |
| Restriction Enzyme Based | restriction fragment length polymorphism (RFLP), restriction site generating PCR |
| Ligation Based | Oligonucleotide ligation assay |
| Other | Invader assay |

* Note: not useful for detection of promoter polymorphisms.

Preferred mutation detection techniques include ARMS™, ALEX™, Taqman™, molecular beacons, RFLP, OLA, restriction site based PCR, FP-TDI, heteroduplex analysis, and fluorescence resonance energy transfer techniques.

Accordingly, this invention includes a number of general uses that can be expressed as follows. This invention provides for the use of any of the nucleic acid segments described above in the diagnosis or monitoring of diseases, such as cancer, inflammation, heart disease, diseases of the CNS, and susceptibility to infection by microorganisms. This invention further provides for the use of any of the nucleic acid segments in the manufacture of a medicament for the treatment (includes, inter alia, preventative (e.g., prophylactic), palliative and curative treatment) of such diseases. This invention further provides for the use of any of the DNA segments as a pharmaceutical in any suitable dosage form.

The present invention is described in full, clear, concise, and exact terms to enable those skilled in the art to make and use this invention. In addition,

EXAMPLES and illustrations are provided but do not limit this invention which is defined by the appendant claims. It will also be understood that certain changes and modifications that may be practiced are within the scope of the appendant claims.

All documents, i.e., publications and patent applications, cited in this disclosure are incorporated by reference herein in their entireties for all purposes to the same extent as if each of the individual documents were specifically and individually indicated to be so incorporated by reference herein in its entirety.

## Claims

1. A nucleic acid segment from the human TACR1 gene of between 10 and 100 nucleotides comprising a fragment selected from the group consisting of CACTCAGCTG (SEQ ID NO:1), CACTCTGCTG (SEQ ID NO:2), GCGGACCGCA (SEQ ID NO:3), GCGGACGGCA (SEQ ID NO:4), TCCCACGGCAA (SEQ ID NO:5), TCCCACTGCAA (SEQ ID NO:6), GCCCAGAAAAAG (SEQ ID NO:7), GCCCATAAAAAG (SEQ ID NO:8), CAGCCACAGGA (SEQ ID NO:9), CAGCGACAGGA (SEQ ID NO:10), TACCGCCTAG (SEQ ID NO:11), TACCGCTTAG (SEQ ID NO:12), CACCCTCGTCC (SEQ ID NO:13), CACCCTCATCC (SEQ ID NO:14), AGTATGGGTTA (SEQ ID NO:15), AGTATGGCTTA (SEQ ID NO:16), TGCTCGCTCTCCA (SEQ ID NO:17), and TGCTCGCACTCCA (SEQ ID NO:18), wherein said fragment comprises a polymorphic site, or the complement of said fragment.

2. A segment as defined in claim 1, wherein said polymorphic site is at position -320 in the 5' region of the human NK1 gene.

3. A segment as defined in claim 1, wherein said polymorphic site is at position -255 in the 5' region of the human NK1 gene.

4. A segment as defined in claim 1, wherein said polymorphic site is at position -176 in the 5' region of the human NK1 gene.

5. A segment as defined in claim 1, wherein said polymorphic site is at position +465 in the 5' UTR region of the human NK1 gene.

6.  A segment as defined in claim 1, wherein said polymorphic site is at position +524 in the 5' UTR region of the human NK1 gene.

7.  A segment as defined in claim 1, wherein said polymorphic site is at position +600 in the 5' UTR region of the human NK1 gene.

8.  A segment as defined in claim 1, wherein said polymorphic site is at position +1722 in exon 5 of the human NK1 gene.

9.  A segment as defined in claim 1, wherein said polymorphic site is at position +1942 in the 3' untranslated region of the human NK1 gene.

10. A segment as defined in claim 1, wherein said polymorphic site is at position -6 (relative to the start of exon 5) in intron 4 of the human NK1 gene.

11. A method of analyzing a nucleic acid, comprising obtaining said nucleic acid from an individual, and determining the base occupying any one of the polymorphic sites selected from the group consisting of CACTCAGCTG (SEQ ID NO:1), CACTCTGCTG (SEQ ID NO:2), GCGGACCGCA (SEQ ID NO:3), GCGGACGGCA (SEQ ID NO:4), TCCCACGGCAA (SEQ ID NO:5), TCCCACTGCAA (SEQ ID NO:6), GCCCAGAAAAAG (SEQ ID NO:7), GCCCATAAAAAG (SEQ ID NO:8), CAGCCACAGGA (SEQ ID NO:9), CAGCGACAGGA (SEQ ID NO:10), TACCGCCTAG (SEQ ID NO:11), TACCGCTTAG (SEQ ID NO:12), CACCCTCGTCC (SEQ ID NO:13), CACCCTCATCC (SEQ ID NO:14), AGTATGGGTTA (SEQ ID NO:15), AGTATGGCTTA (SEQ ID NO:16), TGCTCGCTCTCCA (SEQ ID NO:17), and TGCTCGCACTCCA (SEQ ID NO:18).

12. A method as defined in claim 11, wherein said nucleic acid is obtained from a plurality of said individuals, and said base occupying one of said polymorphic sites is determined in each of said individuals, and further comprising testing each of said individuals for the presence of a disease phenotype, and correlating said presence with said base.

# FIG. 1